**Europäisches Patentamt**

(19) **European Patent Office**

**Office européen des brevets**

(11) Veröffentlichungsnummer: **0 136 262**

**B1**

(12) **EUROPÄISCHE PATENTSCHRIFT**

(45) Veröffentlichungstag der Patentschrift:
**08.04.87**

(21) Anmeldenummer: **84810400.6**

(22) Anmeldetag: **14.08.84**

(51) Int. Cl.⁴: **A 61 B 17/04,** A 61 B 17/28,
A 61 B 17/42

(54) **Chirurgisches Instrument, insbesondere Hysterektomium.**

(30) Priorität: **13.09.83 CH 4972/83**

(43) Veröffentlichungstag der Anmeldung:
**03.04.85 Patentblatt 85/14**

(45) Bekanntmachung des Hinweises auf die Patenterteilung:
**08.04.87 Patentblatt 87/15**

(84) Benannte Vertragsstaaten:
**AT BE DE FR GB IT NL SE**

(56) Entgegenhaltungen:
**WO-A-83/00994**
**CH-A-166 352**
**DE-C-220 179**
**FR-A-2 445 133**
**GB-A-1 158 113**
**US-A-1 577 054**
**US-A-2 286 578**
**US-A-3 946 740**
**US-A-4 164 225**

(73) Patentinhaber: **FRITZ GEGAUF AG BERNINA-
NAEHMASCHINENFABRIK, Seestrasse, CH- 8266
Steckborn (CH)**

(72) Erfinder: **Lahodny, Johann, Conradstrasse 36,
A-3950 Gmünd (AT)**
Erfinder: **Dreier, Ernst, Mühlhofstrasse 27, CH- 8266
Steckborn (CH)**

(74) Vertreter: **Steiner, Martin, c/o AMMANN
PATENTANWAELTE AG BERN Schwarztorstrasse
31, CH- 3001 Bern (CH)**

LIBER, STOCKHOLM 1987

## Beschreibung

Die vorliegende Erfindung betrifft ein chirurgisches Instrument, insbesondere ein Hysterektomium zum Abklemmen, Abbinden und Durchtrennen von Gewebe. Bekannte Instrumentarien umfassen spezialisierte Instrumente wie Hysterektomieklemmen zum Abklemmen von Gewebe (DE-PS 30 13 836), Scheren zum Durchschneiden von Gewebeteilen und Nähgeräte aller Art. Die Arbeit bei der Operation, beispielsweise einer Gebärmutterentfernung, wird dadurch erheblich erschwert, dass die einzelnen Instrumente nacheinander, teils auch miteinander an derselben Stelle zum Einsatz kommen müssen. Besonders der Nähvorgang ist schwierig und zeitraubend, weil seitlich der im Klemmzustand verriegelten Klemme Umstechungen ausgeführt werden müssen. Es bestehen zwar Versuche, kombinierte Geräte zu schaffen, welche mehr als einem Zwecke dienen.

Es ist z.B. eine chirurgische Klemme bekannt, welche die Gewebeteile, insbesondere zu ligierende Teile von Blutgefässen, vorerst mittels Klemmbacken fasst und hält und dann mittels einer integrierten Heftvorrichtung mit je einer Klammer zu versehen gestattet. Dieses Instrument kann aber nicht zugleich zum Schneiden von Gewebeteilen dienen und das Anbringen von Klammern ist nicht in allen Fällen befriedigend oder überhaupt anwendbar (US-PS 2, 853,074).

Es ist weiter eine chirurgische Zange zum Abklemmen und nachträglichen Durchschneiden von Gefässen bekannt (US-PS 3,175,556). Diese Zange gestattet auf abzutrennende und abzuschliessende Gefässe mittels Klemmbacken nebeneinander zwei Klammern aufzuklemmen und dann mittels einer separat betätigbaren Schneidklinge das Gefäss zwischen den beiden aufgepressten Klammern zu trennen. In diesem Falle könnte man von einer dreifachen Funktion, nämlich Klemmen, Abbinden (Ligieren) und Trennen sprechen. Die Anwendung des Instrumentes ist jedoch beschränkt auf das Abschliessen und Trennen von Gefässen. Weder das Instrument noch die anzubringenden Klammern sind dazu geeignet, in einem fortlaufenden Vorgang Gewebeteile abzuklemmen, zu vernähen und zu trennen.

Es ist auch eine chirurgische Zange bekannt, deren Klemmbacken gewellt sind, so dass dünnes Gewebe zwischen denselben wellenförmig verformt wird, derart, dass eine Nähnadel durch Ausnehmungen der Klemmbacken und das gewellte Gewebe durchgeführt werden kann, um den geklemmten Gewebeabschnitt zu vernähen. (CH-PS 166 352). Es ist ferner ein längs der Klemmbacken verschiebbares Messer zum Durchschneiden des Gewebes vorgesehen. Das Instrument besitzt nur Handgriffe zur Betätigung der Klemmbacken, was voraussetzt, dass man zum Nähen und Schneiden von Hand im Bereiche der Klemmbacken arbeiten muss.

Es ist auch ein Hilfsgerät zum chirurgischen Nähen bekannt, welches eine Reihe von Nadeln aufweist, welche zum gleichzeitigen Einziehen mehrerer Fäden dient, von welchen jeder in eine Nadel eingezogen ist. (US-PS 1 577 054).

Ziel der vorliegenden Erfindung ist es, durch eine neuartige, besonders vorteilhafte Kombination von Funktionen in einem Instrument ein Arbeiten je auf einem längeren Abschnitt in einem Arbeitszyklus zu ermöglichen ohne im Bereiche der Klemmbacken zu arbeiten und dadurch die Operation entscheidend zu vereinfachen, sicherer zu gestalten (keine Nachblutungen) und zu beschleunigen. Ein Instrument dieser Art ist in Anspruch 1 umschrieben.

Es handelt sich also um ein Instrument oder Hysterektomium mit integrierter Klemm-, Trenn- und Nähvorrichtung, welches derartige fortlaufende Abklemm-, Näh- und Trennvorgänge erlaubt, wie sie beispielsweise zur rationellen Durchführung von Gebärmutterentfernungen erforderlich sind, wobei das Gewebe zwischen Gebärmutter und Beckenwand (Parametrium) zusammengeklemmt und gehalten wird, um die darin verlaufenden Blutgefässe sicher abzuklemmen, worauf das beckenseitige Parametrium in raumsparender Weise, ohne den nahegelegten Ureter (Harnleiter) zu verletzen vernäht und schliesslich das Gewebe seitlich der Klemme auf der Gebärmutterseite abgetrennt wird. Dabei werden die besonderen Schwierigkeiten berücksichtigt, welche sich bei Operationen im Körperinnern stellen (stark beengtes Operationsfeld, mangelnde Bewegungsfreiheit und Sicht, etc.). Erfindungsgemäss wird dieses Ziel mittels eines Instrumentes (Hysterektomium) erreicht, das ausser Klemmbacken zum Klemmen eine Schneidklinge zum Trennen der Gewebeteile und eine Nähvorrichtung zum Nähen geklemmter Gewebeteile aufweist. Mittels dieses Hysterektomiums können nun Abschnitte des Gewebes ohne Wechsel des Instrumentes sozusagen in einem Arbeitsgang abschnittweise abgeklemmt, genäht und abgetrennt werden. Vorzugsweise ist hierbei die bewegliche Schneidklinge auf der einen und die Nähvorrichtung auf der andern Seite der Klemmbacken angeordnet. Damit kann bei gleichbleibender Klemmung jeweils auf der einen Seite genäht und auf der andern Seite getrennt werden. Es können mehrere, vorzugsweise in einer Reihe hintereinander angeordnete Nadeln und ein der Fadenverschlingung nacheinander bei allen Nadeln dienender Greiferhaken vorhanden sein, so dass stets ein der Klemmenlänge entsprechender Abschnitt zugleich geklemmt, genäht und getrennt werden kann. Zur weiteren Rationalisierung des Vorganges kann je ein Satz von Nadeln auswechselbar in den einen Arm eines Nähhebels eingesetzt sein, so dass je nach einem erfolgten Klemm-, Näh- und Trennvorgang der

alte Nadelsatz durch einen neuen ersetzt werden kann, in welchen bereits ein Faden eingezogen ist, worauf sogleich weitergearbeitet werden kann.

Anhand eines speziell für die oben bereits erwähnte Gebärmutterentfernung konzipierten Ausführungsbeispiels des Instrumentes oder Hysterektomiums, das in der Zeichnung dargestellt ist, wird nun die Erfindung näher erläutert.

Figur 1 ist eine Seitenansicht des Hysterektomiums,

Figur 2 zeigt eine Draufsicht auf dasselbe, mit einer Übersicht über das Operationsfeld bei einer ersten Operationsart,

Figur 3 ist eine der Figur 2 entsprechende Darstellung für eine zweite Operationsart,

Figuren 4 bis 9 zeigen verschiedene Stadien des Nähvorgangs,

Figuren 10 und 11 zeigen eine vergrösserte Draufsicht und Seitenansicht einer Nadel,

Figuren 12 und 13 zeigen einen auswechselbaren Nadelsatz in Seitenansicht und Draufsicht,

Figur 14 zeigt einen Querschnitt durch den Nadelsatz und seine Halterung im Nähhebel,

Figur 15 zeigt eine vergrösserte Draufsicht mit dem angebauten Teil der Nähvorrichtung und den Nadeln im Schnitt, und

Figur 16 zeigt einen Schnitt nach Linie XVI-XVI in Figur 15.

Das dargestellte Hysterektomium weist eine untere Klemmbacke 1 und eine obere Klemmbacke 2 auf, die mit je einem Hebel (Branche) 3, bzw. 4 fest verbunden sind. Diese Hebel sind mittels einer Achse 5 schwenkbar miteinander verbunden und weisen an den Enden Ringgriffe 6, bzw. 7 auf. Beide Arme 3 und 4 sind mit je einer Verriegelungsnase 8, bzw. 9 mit einer Rast- oder Verriegelungsverzahnung versehen. Sie dienen dazu, die Klemme in zusammengedrücktem Zustand selbsttätig zu verriegeln und damit dem Chirurgen weitere Manipulationen zu gestatten, bzw. zu erleichtern. Soweit entspricht die Klemme weitgehend bekannten Klemmen.

Auf der Achse 5 ist auch der Betätigungshebel 10 einer Schneidklinge 11 gelagert, der an dem der Schneidklinge 11 gegenüberliegenden Ende mit einem Fingergriff 12 versehen ist, unter welchen zur Betätigung der Schneidvorrichtung gegriffen werden kann. Der Fingergriff 12 greift unter den Betätigungshebel 4 der oberen Klemmbakke, die damit einen Anschlag für diesen Fingergriff und die dadurch betätigbare Schneidklinge bildet.

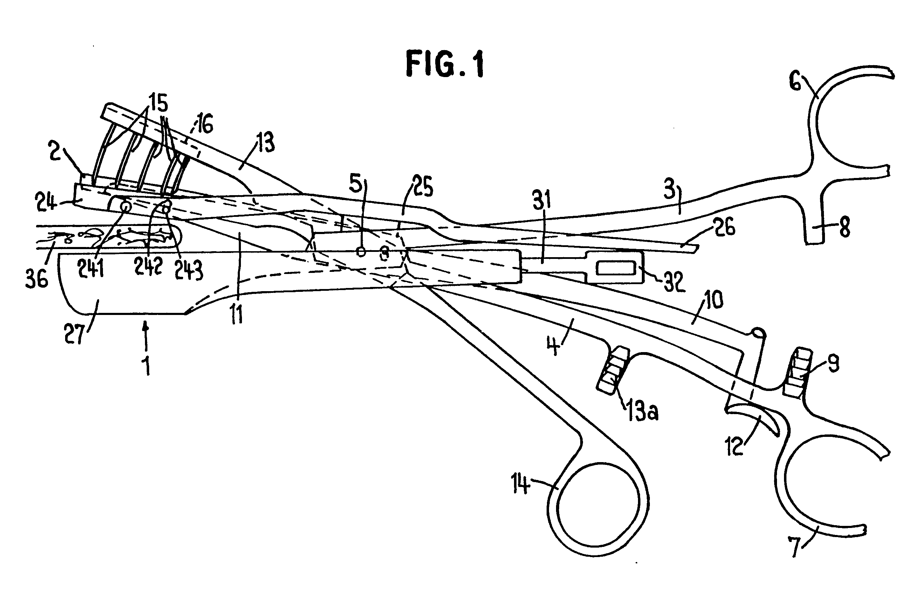

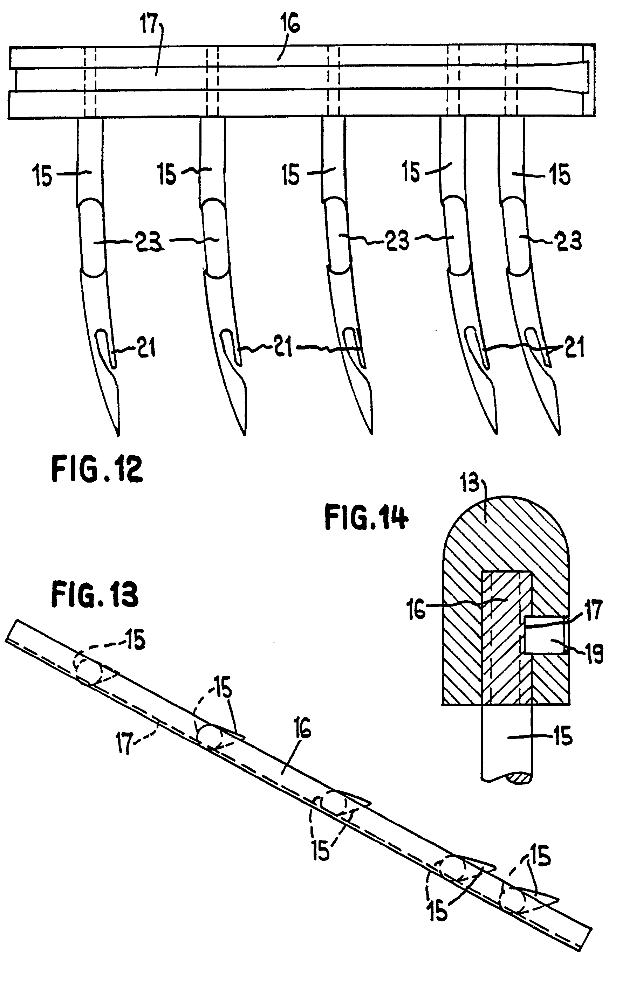

Auf der Achse 5 ist ebenfalls ein Nähhebel 13 mit einem Ringgriff 14 schwenkbar gelagert. Das dem Ringgriff 14 gegenüberliegende Ende des Nähhebels 13 trägt einen Satz von in Figur 1 schematisch angedeuteten Nähnadeln 15. Die Ausbildung dieses Nadelsatzes und seine Halterung im Näharm ist in den Figuren 12 bis 14 ausführlicher dargestellt. Die Nadeln 15 sitzen in einem flachen Nadelträger 16, der einseitig eine Nut 17 aufweist. Die Nut 17 ist am inneren Ende des Trägers 16 etwas erweitert, um das Einschieben des Nadelträgers 16 zu erleichtern. Dieses Einschieben erfolgt in einen Schlitz 18 am vorderen Ende des Nähhebels 13, in welchen Schlitz seitlich zwei oder mehr Bolzen 19 einragen. Durch diese in die Nut 17 eingreifenden Bolzen ist der Nadelträger 16 im Schlitz 18, bzw. im Näharm 13 zuverlässig gehalten und doch kann der Nadelträger mit den Nadeln ohne weiteres nach vorne herausgezogen und durch einen anderen ersetzt werden. Der Nadelträger 16 ist in Längsrichtung leicht gebogen ausgeführt, so dass er elastisch gegen die Wandungen der Nut 17 anliegt und in derselben gesichert ist. Es sind auch andere Befestigungsarten denkbar, z.B. durch Schnappverbindung.

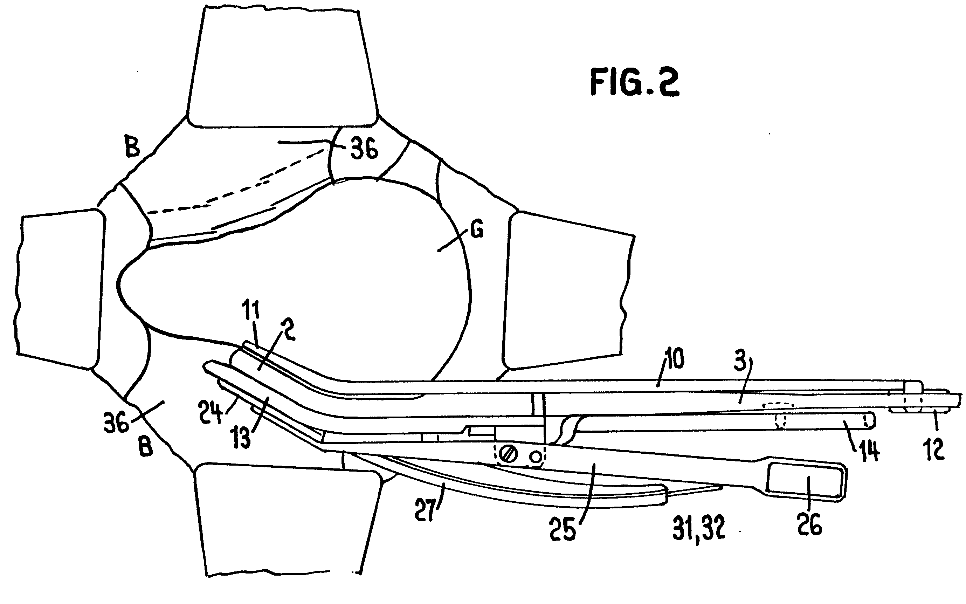

Wenigstens die innersten zwei der vorzugsweise fünf vorhandenen Nadeln 15 stehen enger beisammen als die übrigen Nadeln. Der Grund hierzu wird noch erläutert. Die Nadeln sind gekrümmt, wobei je das Zentrum ihrer Krümmung mit der Achse 5 zusammenfällt, im übrigen liegen die Nadeln, wie insbesondere Figur 13 zeigt, in Radialebenen bezüglich der Achse 5. Unter diesen Umständen dringen die Nadeln bei der Verschwenkung des Nadelhebels 13 ohne seitliche Relativbewegung bezüglich der Klemmbacken in zwischen den Klemmbacken gehaltene Gewebeteile ein, ohne grössere Verletzungen dieses Gewebes als unbedingt erforderlich zu verursachen. Die Klemmbacken 1 und 2, der wirksame Teil der Schneidklinge 11, das mit Nadeln besetzte Ende des Nähhebels 13 und weitere, noch zu beschreibende Teile sind gegenüber den Betätigungshebeln, um 20 bis 30° abgewinkelt, was aus noch zu erläuternden Gründen die Zange ganz besonders für ihren spezifischen Zweck geeignet macht. Mit anderen Worten bilden die Klemmbacken mit ihren Betätigungshebeln einen stumpfen Winkel bezüglich welchem die Schneidklinge 11 innen und die Näheinrichtung mit den Nadeln 15 aussen liegt (Figur 2).

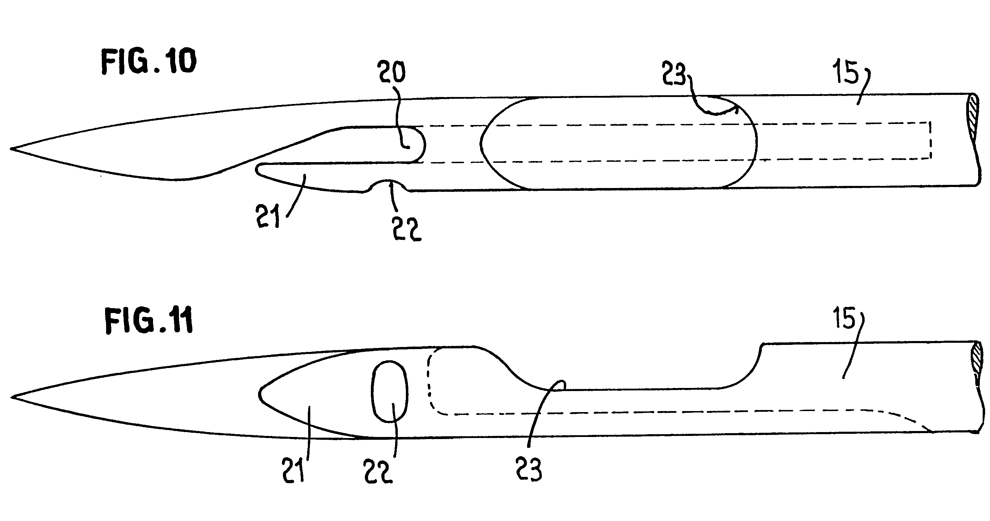

Die besondere Ausbildung der im Ausführungsbeispiel verwendeten Nähnadeln ist in den Figuren 10 und 11 dargestellt. Die Nadeln weisen hinter ihrer Spitze dreieckigen Querschnitts offene Fadenführungsöhre 20 auf. Die Elastizität der Zunge 21, welche die Öffnung der Fadenführungsöhre verengt, ist durch eine geschwächte Stelle 22 erhöht. In einem hinter dem Öhr 20 liegenden Bereich ist der Nadelschaft mit einer Ausnehmung 23 versehen, welche etwa den halben Querschnitt des Nadelschaftes belässt.

Dem Nadelsatz ist ein Fadenspanner 24 zugeordnet, dessen Betätigungshebel 25 um zwei rechtwinklig zueinander stehende Achsen schwenkbar ist und somit eine Schwenkbewegung im Sinne der bisher beschriebenen Hebel sowie eine Schwenkbewegung zur seitlichen Entfernung des Fadenspanners aus dem Bereich des Nadelsatzes

ausführen kann. Die Betätigung erfolgt hierbei an der Betätigungstaste 26 am hinteren Ende des Hebels 25. Der Fadenspanner 24 ist mittels eines Stiftes 241 schwenkbar an seinem Betätigungshebel 25 aufgehängt, wobei die Schwenkbewegung durch einen in ein Langloch 242 greifenden Stift 243 begrenzt ist.

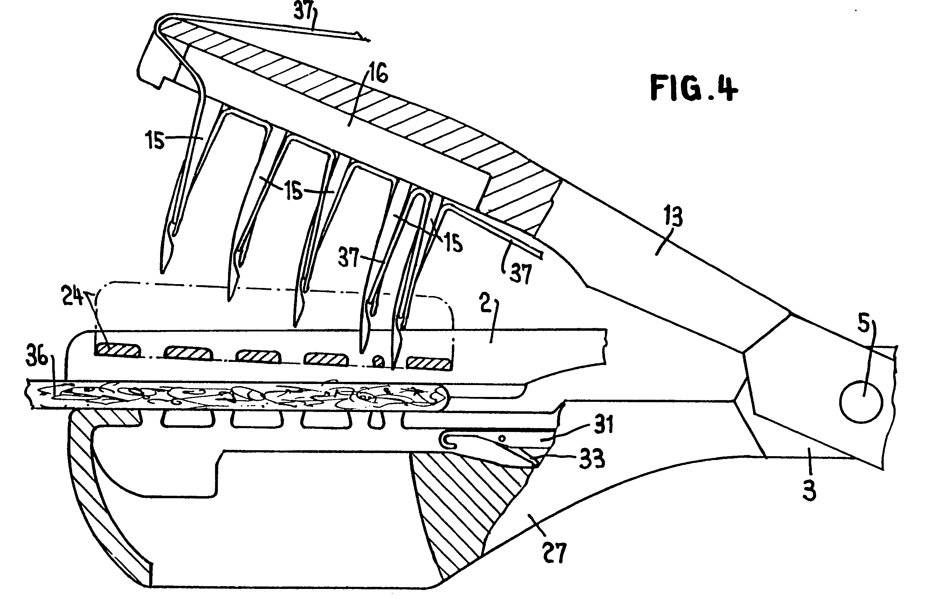

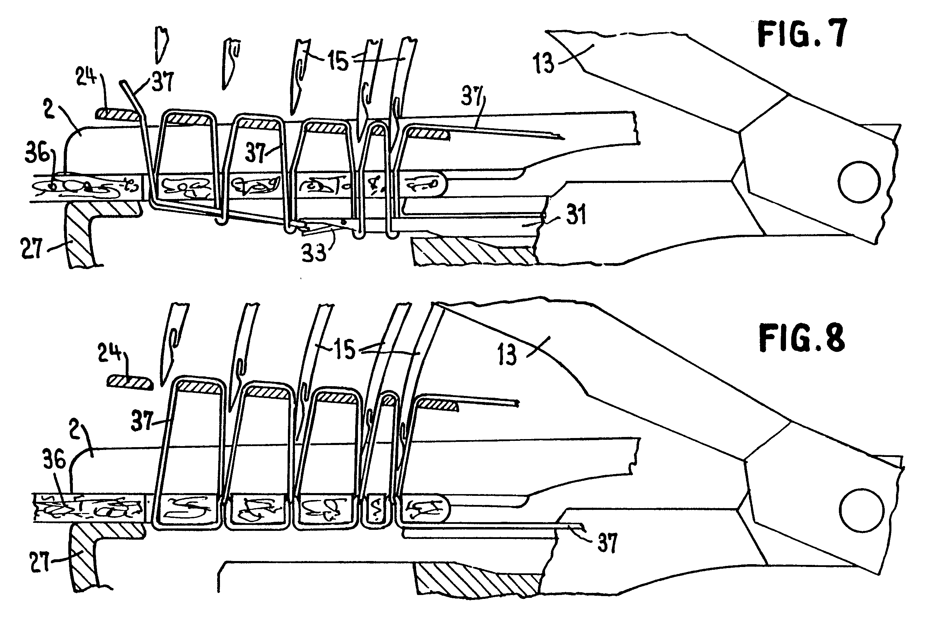

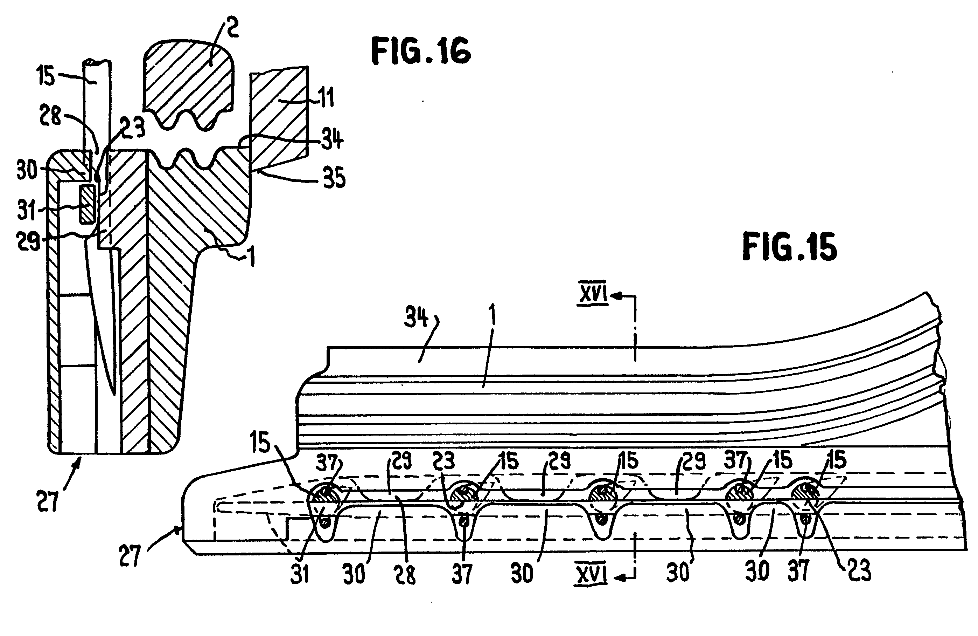

Auf der der Schneidklinge 11 gegenüberliegenden Seite ist an der unteren Klemmbacke ein Kasten 27 (insbesondere Figuren 15 und 16) angebaut, in welchen die Nadeln nach dem Durchstechen des Gewebes eindringen können, wie die Figuren 15 und 16 andeuten. Der Kasten 27 ist zweiteilig ausgebildet und die Ränder der oberen Wandungen dieser Kastenteile sind gemäss Figur 15 derart geformt, dass sich eine schlitzartige zusammenhängende Öffnung 28 ergibt. Diese Öffnung 28 weist erweiterte Stellen auf, wo die Nadeln 15 durch die Öffnung in den Kasten eindringen. Durch seitliche Nocken 29 des einen und übergreifende Nocken 30 des anderen Kastenteils bilden dieselben eine Längsführung für einen Greiferhaken 31, der gemäss Figuren 1 bis 3 hinten aus dem Kasten 27 herausragt und mit einem Betätigungsgriff 32 versehen ist. Der Greiferhaken 31 ist elastisch vorgespannt, so dass er gegen die Nocken 29 anliegt, jedoch unter der Wirkung der in den Kasten eindringenden Nadeln in den freien Raum nach links in Figur 16 ausweichen kann. Eine Vorbereitungsposition des Greiferhakens 31 gemäss Figur 4 ist durch eine nicht dargestellte Rast bestimmt. Wie aus den Figuren 4 bis 7 ersichtlich, weist der Greiferhaken eine bewegliche Klappe 33 auf, welche sich beim Einschieben des Greiferhakens, gemäss Figur 4, zurücklegt und beim Zurückziehen des Greiferhakens, gemäss Figur 7, nach vorne gelegt wird, um die Öse des Greiferhakens zu schliessen. Wie Figur 16 zeigt, befindet sich der Greiferhaken bei voll in den Kasten 27 eingeführten Nadeln 15 im Bereiche der Ausnehmungen 23 der Nadeln 15, so dass die eingeführten Nadeln eine Längsverschiebung des Greiferhakens 31 kaum behindern.

Die Figuren 15 und 16 zeigen auch die besondere Ausführung der Klemmbacken mit gewellten Klemmflächen zur Erhöhung des Haltes des geklemmten Gewebes. Die eine Seitenkante 34 der unteren Klemmbacke ist als Schnittkante geschliffen, welche mit der Schnittkante 35 der Schneidklinge 11 zusammenwirkt.

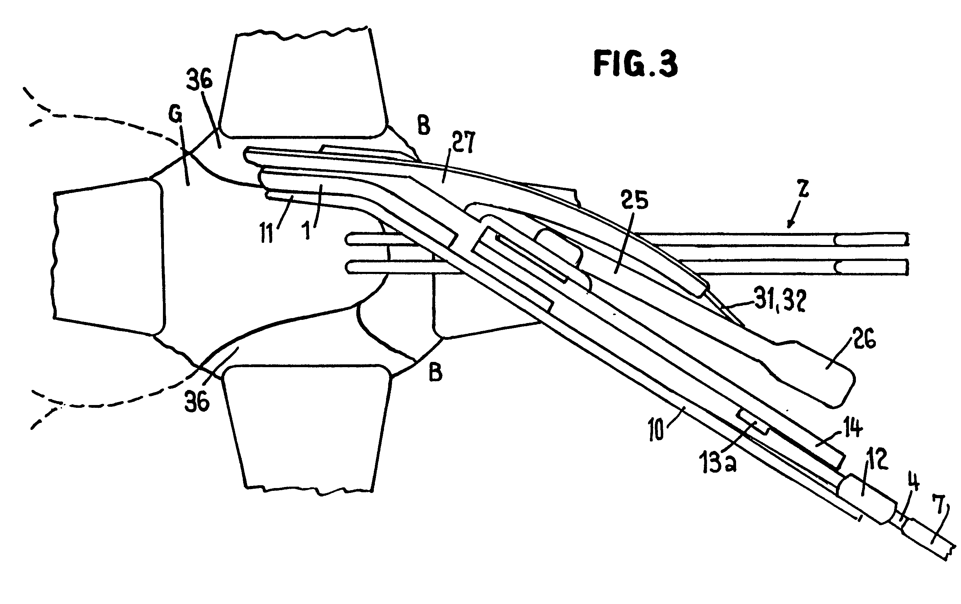

Das Vorgehen bei einer Operation, insbesondere Gebärmutterentfernung, ist oben schon kurz angedeutet worden. In den Figuren 2 und 3 ist angedeutet, dass das Hysterektomium in jedem Fall benützt werden kann, ob es sich nun um die Operation von abdominal (einem Bauchschnitt) oder (Fig. 2) vaginal (von der Scheide aus) (Fig. 3) handelt. In beiden Fällen ist mittels Blättern das Parametrium eingestellt. Bei der Operation nach Fig. 3 wird zudem eine Zange Z benützt, mit welcher die Gebärmutter G in die gewünschte Position gebracht wird. Zu Beginn befindet sich die Klemme im offenen Zustand nach Figur 1, d.h. die Klemmbacken sind geöffnet und der Nadelsatz 15 mit dem Fadenspanner 24 sind aus dem Bereich der Klemmbacken ausgeschwenkt und der Greiferhaken befindet sich in Vorbereitungsstellung (Fig. 4). Das Gewebe (Parametrium) zwischen Gebärmutter G und Beckenwand B, das in den Figuren mit 36 bezeichnet ist, kann nun nach Einschieben der offenen Klemme von einer Seite her durch Zusammendrücken der Klemmbacken 1 und 2 mittels der Ringgriffe 6 und 7 auf einem ersten Abschnitt erfasst und zusammengeklemmt werden, derart, dass die darin verlaufenden Blutgefässe sicher abgeklemmt sind. Das in Figur 16 dargestellte gewellte Profil der Klemmflächen der Klemmbacken verbessert hierbei den Halt des Gewebes zwischen den Klemmbacken. Dieselben werden im zusammengeklemmten Zustand durch Eingriff der Verriegelungsverzahnung der Nasen 8 und 9 ohne weiteres Dazutun des Chirurgen gehalten. Nun kann das Gewebe seitlich der Klemmbakken auf der Beckenwandseite durch Betätigung der Nähvorrichtung ohne jegliche Raffung oder Ureterirritation vernäht werden.

Der Nähvorgang wird anhand der Figuren 4 bis 9 erläutert. Figur 4 zeigt dabei die Ausgangsstellung. In den im Nähhebel 13 befindlichen Nadelsatz ist ein Nähfaden 37 eingezogen und zwar ist dieser Faden durch jedes Nadelöhr durchgeführt und am Träger 16 des Nadelsatzes in geeigneter Weise angeheftet. Die freien Enden des Fadens sind lang genug, um das Verknüpfen des Fadens am Ende des Nähvorgangs zu ermöglichen. Im vorderen Teil weisen die Nadeln einen dreieckigen Querschnitt auf, um die beim Nähen entstehenden Gewebeperforationen auf ein Minimum zu beschränken. Der Nadelsatz wird nun durch Betätigen des Nähhebels 13 mittels des Ringgriffes 14 durch den Fadenspanner 24 und das Gewebe 36 hindurch in den Kasten 27 verschwenkt. In diesem Augenblick befindet sich der Greiferhaken noch in der Figur 4 dargestellten Vorbereitungsstellung und behindert das Eintreten der Nadeln in den Kasten 27 nicht. Der Nähhebel 13 und der Nadelsatz verbleiben dann selbsttätig in dieser Stellung, indem der Nähhebel 13 an einer Verriegelungsverzahnung 13a des Hebels 4 der oberen Klemmbacke verriegelt wird.

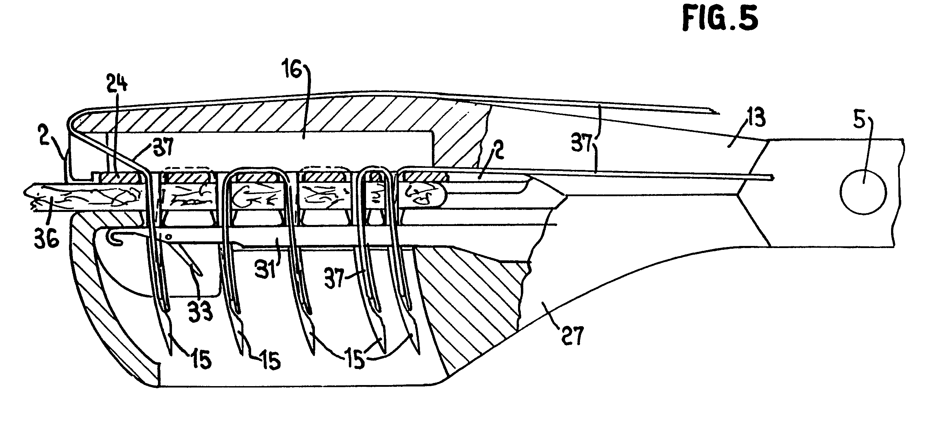

Figur 5 zeigt ein nächstes Stadium des Nähvorganges. Die Nadeln sind voll in den Kasten 27 eingeschwenkt und der Nähhebel 13, bzw. der in denselben eingesetzte Nadelsatz drückt den Fadenspanner 24 dank seiner Schwenkbarkeit gleichmässig auf das Gewebe 36. In diesem Stadium kann nun der Greiferhaken 31 unmittelbar neben den Nadeln hindurch in die in Figur 5 dargestellte vordere Endlage vorgeschoben werden und zwar mittels seines Griffes 32. Die Enden des Nähfadens 37 sind nach hinten gelegt. Nun werden der Nadelsatz und der

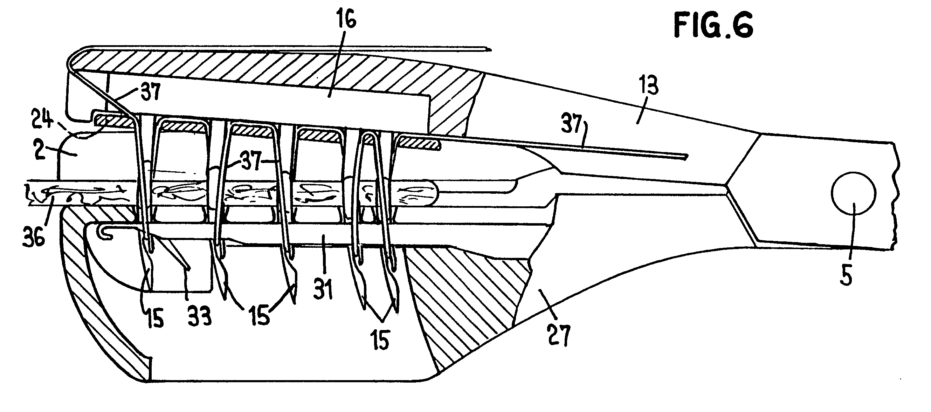

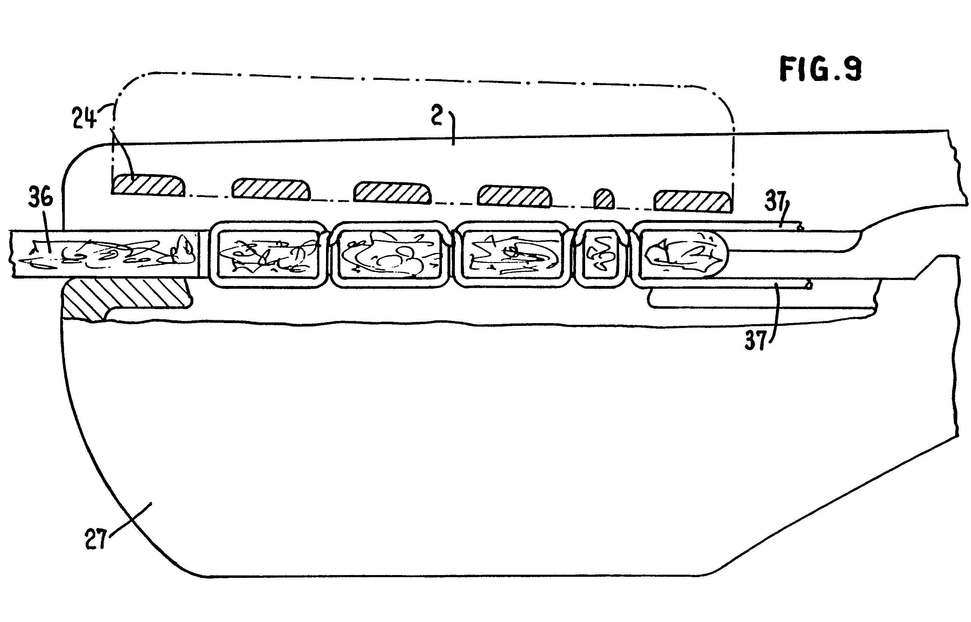

Fadenspanner 24 etwas angehoben in die in Figur 6 dargestellte Position, wobei sich die Fadenschlingen um den elastisch seitlich ausweichenden Greiferhaken legen, d.h. satt an der Unterseite des Greiferhakens anliegen. Der Greiferhaken wird dann zurückgezogen, wie Figur 7 zeigt, wobei die vorderste Fadenschlinge vom Greiferhaken erfasst wird. Spätestens beim Anlaufen der Klappe 33 gegen die Fadenschlinge der zweiten Nadel wird diese Kappe nach vorne gelegt und bildet zusammen mit dem Haken ein geschlossenes Öhr, in welchem nun die vorderste Fadenschlinge gehalten ist, welches jedoch die weiteren Fadenschlingen nicht erfasst. Die vorderste Fadenschlinge, bzw. das beim Zurückziehen des Greiferhakens mitgenommene Fadenende wird somit durch die übrigen Fadenschlingen durchgezogen, wie Figur 7 andeutet, wobei für diesen Vorgang der Nadelsatz vorzugsweise in seine ausgerückte unwirksame Stellung gebracht wird. Der hiermit freigegebene Greiferhaken 31 geht federnd in seine Ausgangslage an den Nocken 29 zurück (Fig. 16). Er wird schliesslich ganz nach hinten herausgezogen. Nun wird der Fadenspanner 24 gemäss Figur 8 angehoben und zieht dabei den unter dem Gewebe verlaufenden Faden soweit in das Gewebe ein, dass die Verschlingungen zwischen oberem und unterem Fadenteil auf die Oberseite des zusammengepressten Gewebes 36 zu liegen kommen. Dank der Schwenkbarkeit des Fadenspanners erfolgt ein gleichmässiges Spannen aller Schlingen. Dann wird das obere Fadenende festgezogen, bis der Fadenspanner auf dem Gewebe aufliegt. Hierauf wird der Fadenspanner um seine zweite Schwenkachse seitlich ausgeschwenkt, und die nur noch kurzen freiliegenden oberen Fadenschlingen können ohne Verwicklungsgefahr durch Ziehen am Fadenende festgezogen werden, womit die Situation nach Figur 9 entsteht. Die Perforationen im Gewebe sind durch die Fadenknoten verschlossen. Hierauf werden die beiden Fadenenden verknotet, womit das abgetrennte Parametrium beckenwandseitig auf einem gewissen Abschnitt fest vernäht ist. Das Parametrium wird nun auf der Gebärmutterseite durch Betätigung der Schneidklinge 11 abgetrennt. Es werden nun auch die Klemmbacken durch seitliches Spreizen der Arme 3 und 4 und damit Ausrücken der Verriegelungsverzahnung an den Nasen 8 und 9 geöffnet, womit die Klemme für einen weiteren Näh- und Trennvorgang an einem nächsten Abschnitt frei wird. Der alte Nadelsatz 15 wird nach vorne herausgezogen, und es wird ein neuer, bereits mit Nähfaden versehener Nadelsatz eingeschoben. Der Greiferhaken wird wieder in die Vorbereitungsstellung (Fig. 4) eingeschoben. Damit ist das Instrument bereit zum Klemmen, Vernähen und Trennen des nächsten Abschnittes. Diese Vorgänge werden wiederholt, bis das ganze Parametrium abgetrennt und vernäht ist. Wie in Figur 2 oben angedeutet, können dabei die Abschnitte der Naht und des Schnittes leicht versetzt und überlappend ausgeführt werden.

Wie Figur 7 zeigt, wird die vorderste Fadenschlinge, bzw. das an dieselbe anschliessende Fadenende durch den Greiferhaken unter der Abdeckung des Kastens 27 durchgezogen. Die oben beschriebene, durchgehende Öffnung 28 gestattet es jedoch, mit dem Faden nach oben frei auszufahren, d.h. es ergeben sich keinerlei Schwierigkeiten mit der Fadenführung. Der Grund, weshalb die zwei hintersten Nadeln in kleineren Abständen angeordnet sind als die übrigen Nadeln liegt darin, dass durch die engeren Stiche an demjenigen Ende des Nahtabschnittes, an dem die Fadenenden verknpüft sind, ein selbständiges Lösen der Naht erschwert werden soll. Man könnte gegebenenfalls auch ohne ein Verknüpfen der Fadenenden auskommen, besonders, wenn mehr als zwei engstehende Nadeln vorhanden sind.

Die Erfindung ist keineswegs auf die dargestellte Ausführungsform beschränkt. Für andere Operationen können Einzelheiten des Instrumentes anders ausgeführt sein. Insbesondere kann der Nadelsatz mehr oder weniger Nadeln aufweisen und länger oder kürzer sein, wobei natürlich der Länge durch die aufzuwendenden Kräfte Grenzen gesetzt sind. Die Nadeln können anstelle einer verengten Eintrittsöffnung in das Öhr 20 elastische Schliessklappen für das Öhr aufweisen. Die aktiven Teile der Klemme können überhaupt nicht oder in einer anderen Richtung abgekröpft sein gegenüber den Betätigungshebeln.

**Patentansprüche**

1. Hysterektomium in Form eines Mehrzweckinstrumentes, mit Klemmbacken und einer Verriegelungsvorrichtung zum Arretieren der Klemmbacken in Klemmstellung, dadurch gekennzeichnet, dass auf der einen Seite der Klemmbacken (1, 2) eine Näheinrichtung (13 - 15, 24, 31) angeordnet ist, um geklemmte Gewebeteile auf einem Abschnitt der Länge der Klemmbacken zu vernähen, und dass auf der andern Seite der Klemmbacken (1, 2) eine bewegliche Schneidklinge (11) vorgesehen ist, die aus einer unwirksamen Offenstellung auf der Seite der einen Klemmbacke (2) in den Bereich einer Gegenschneidkante (34) an der anderen Klemmbacke (1) bewegbar ist, um geklemmtes Gewebe auf einem Abschnitt der Länge der Klemmbacken seitlich derselben durchzuschneiden.

2. Hysterektomium nach Anspruch 1, dadurch gekennzeichnet, dass die Schnittkante der Schneidklinge (11) bei unwirksamer Stellung derselben geschützt an der Seite der einen Klemmbacke (2) anliegt.

3. Hysterektomium nach Anspruch 1, wobei die Klemmbacken (1, 2) mit ihren schwenkbar verbundenen Betätigungsarmen (3, 4) einen stumpfen Winkel einschliessen, dadurch gekennzeichnet, dass bezogen auf den Winkel die Schneidklinge (11) innen und die Näheinrichtung (13-15, 24, 31) aussen angeordnet ist.

4. Hysterektomium nach einem der Ansprüche 1 oder 3, dadurch gekennzeichnet, dass die Näheinrichtung mehrere Nadeln (15) und einen der Fadenverschlingung dienenden Greiferhaken (31) aufweist.

5. Hysterektomium nach Anspruch 4, dadurch gekennzeichnet, dass die Näheinrichtung einen separat betätigbaren, in Stichrichtung der Nadeln (15) und quer dazu bewegbaren Fadenspanner (24) aufweist.

6. Hysterektomium nach Anspruch 5, dadurch gekennzeichnet, dass der Fadenspanner (24) schwenkbar mit seinem Betätigungshebel (25) verbunden ist, um ein gleichmässiges Spannen des Fadens zu erzielen.

7. Hysterektomium nach einem der Ansprüche 4 oder 5, dadurch gekennzeichnet, dass ein Nadelsatz (15, 16) auswechselbar in den einen Arm eines Nähhebels eingesetzt ist.

8. Hysterektomium nach Anspruch 7, dadurch gekennzeichnet, dass der Nadelsatz (15, 16) mit elastischer Vorspannung im Nähhebel gehalten ist.

9. Hysterektomium nach einem der Ansprüche 4 bis 7, dadurch gekennzeichnet, dass an der Seite der einen Klemmbacke (1) ein Kasten (27) vorgesehen ist, in welchen die Nadeln (15) durch eine durchbrochene Abdeckung eintreten können und in dem der Greiferhaken (31) verschiebbar geführt ist.

10. Hysterektomium nach Anspruch 9, dadurch gekennzeichnet, dass der Greiferhaken (31) elastisch seitlich gegen die Nadeln anliegt und quer zu denselben ausweichen kann.

11. Hysterektomium nach Anspruch 9 oder 10, dadurch gekennzeichnet, dass die Abdeckung eine Öffnung (28) mit Erweiterungen an den Durchtrittstellen je einer Nadel (15) aufweist.

12. Hysterektomium nach einem der Ansprüche 1 oder 3 bis 11, dadurch gekennzeichnet, dass die Nadeln (15) bezüglich der Schwenkachse (5) des Nähhebels (13) in Radialebenen liegen und eine Krümmung mit Zentrum in der Schwenkachse aufweisen.

13. Hysterektomium nach einem der Ansprüche 1 oder 3 bis 12, dadurch gekennzeichnet, dass die Nadeln (15) ein offenes Öhr mit verengter Öffnung aufweisen.

**Claims**

1. Hysterectome in form of a polyvalent instrument with jaws and a locking device for stopping the jaws in a clamped position, characterized in that a sewing installation (13-15, 24, 31) is arranged on one side of the jaws (1, 2) in order to sew parts of clamped tissue over a section of the length of the jaws and in that, on the other side of the jaws (1, 2) there is provided a movable cutting blade (11) which is capable to be moved from an ineffective open position on the side of one of the jaws (2) to the reach of a counter-cutting edge (34) of the other jaw (1), in order to cut off clamped tissue over a section of the length of the jaws, at the side thereof.

2. Hysterectome according to claim 1, characterized in that the cutting edge of the cutting blade (11) is protected when lying in its ineffective position at the side of one of the jaws (2).

3. Hysterectome according to claim 1 in which the jaws (1, 2) enclose an obtuse angle with their slewably connected actuating arms (3, 4), characterized in that with respect to said angle, the cutting blade (11) is arranged internally and the sewing installation (13-15, 24, 31) externally.

4. Hysterectome according to one of the claims 1 to 3, characterized in that the sewing installation comprises many needles (15) and a hook (31) for catching and looping the thread.

5. Hysterectome according to claim 4, characterized in that the sewing installation comprises a thread tightener (24) capable to be separately actuated in the direction of the needles stitch and in a direction perpendicular thereto.

6. Hysterectome according to claim 5, characterized in that the thread tightener (24) is pivotably connected to its actuating lever (25) in order to achieve a uniform tension of the thread.

7. Hysterectome according to one of the claims 4 or 5, characterized in that a set of needles (15, 16) is interchangeably set in the arm of a sewing lever.

8. Hysterectome according to claim 7, characterized in that the set of needles (15, 16) is held with an elastic initial tension in the sewing lever.

9. Hysterectome according to one of the claims 4 to 7, characterized in that a case (27) is provided at one side of one of the jaws (1) in which the needles (15) may enter through a pierced cover and in which the hook (31) is movably guided.

10. Hysterectome according to claim 9, characterized in that the hook (31) rests resiliently on the side against the needles and in that it may swerve at right angle thereto.

11. Hysterectome according to claim 9 or 10, characterized in that the cover comprises an opening (28) with widenings at the point of passage of each needle (15).

12. Hysterectome according to one of the claims 1 or 3 to 11, characterized in that the needles (15) are located in radial planes with respect to the swivelling axle (5) of the sewing lever (13) and in that they comprise a curvature the center of which being in the swivelling axle.

13. Hysterectome according to one of the claims 1 or 3 to 12, characterized in that the needles (15) comprise an open eye with a narrowed opening.

**Revendications**

1. Hystérectome sous la forme d'un instrument polyvalent, avec des becs de serrage et un dispositif de verrouillage pour arrêter les becs de serrage en position de serrage, caractérisé en ce qu'une installation de couture (13-15, 24, 31) est disposée sur un côté des becs de serrage (1, 2) pour coudre des parties de tissu pincées sur une partie de la longueur des becs de serrage et en ce que de l'autre côté des becs de serrage (1, 2) est prévue une lame coupante (11) mobile susceptible d'être déplacée à partir d'une position ouverte ineffective sur le côté de l'un des becs de serrage (2) jusque dans le domaine d'une contre-arête coupante (34) de l'autre bec de serrage (1), afin de couper du tissu pincé sur une partie de la longueur des becs de serrage, sur le côté de ceux-ci.

2. Hystérectome selon la revendication 1, caractérisé en ce que l'arête coupante de la lame coupante (11) est protégée lorsqu'elle se trouve dans sa position ineffective au côté de l'un des becs de serrage (2).

3. Hystérectome selon la revendication 1, dans lequel les becs de serrage (1, 2) font un angle obtus avec leurs bras de manipulation (3, 4) connectés de manière pivotante, caractérisé en ce que par rapport à l'angle, la lame coupante (11) est disposée à l'intérieur et l'installation de couture (13-15, 24, 31) à l'extérieur.

4. Hystérectome selon l'une des revendications 1 ou 3, caractérisé en ce que l'installation de couture comprend plusieurs aiguilles (15) et un crochet servant à attraper et à boucler le fil.

5. Hystérectome selon la revendication 4, caractérisé en ce que l'installation de couture comprend un tendeur de fil (24) susceptible d'être actionné séparément en direction du point de couture des aiguilles (15) et dans la direction perpendiculaire.

6. Hystérectome selon la revendication 5, caractérisé en ce que le tendeur de fil (24) est lié de manière pivotante à son levier d'actionnement (25) pour obtenir une tension régulière du fil.

7. Hystérectome selon l'une des revendications 4 ou 5, caractérisé en ce qu'un jeu d'aiguilles (15, 16) est logé de manière interchangeable dans un bras d'un levier de couture.

8. Hystérectome selon la revendication 7, caractérisé en ce que le jeu d'aiguilles (15, 16) est tenu avec une précontrainte élastique dans le levier de couture.

9. Hystérectome selon l'une des revendications 4 à 7, caractérisé en ce qu'une boîte (27) est prévue au côté de l'un des becs de serrage (1), dans laquelle les aiguilles (15) peuvent pénétrer à travers un couvercle ajouré et dans laquelle le crochet (31) est guidé lors de son déplacement.

10. Hystérectome selon la revendication 9, caractérisé en ce que le crochet (31) s'appuie élastiquement de côté contre les aiguilles et en ce qu'il peut dévier perpendiculairement à celles-ci.

11. Hystérectome selon la revendication 9 ou 10, caractérisé en ce que le couvercle comprend une ouverture (28) avec élargissements aux endroits de passage de chaque aiguille (15).

12. Hystérectome selon l'une des revendications 1 ou 3 à 11, caractérisé en ce que les aiguilles (15) se trouvent dans des plans radiaux par rapport à l'axe de pivotement (5) du levier de couture (13) et qu'elles comprennent une courbure dont le centre est sur l'axe de pivotement.

13. Hystérectome selon l'une des revendications 1 ou 3 à 12, caractérisé en ce que les aiguilles (15) comprennent un chas ouvert avec une ouverture étroite.

FIG.1

FIG.2

FIG.3

Z

25

27

B

B

31,32

26

14

12

4

7

13a

10

1

11

36

36

G

FIG.4

0 136 262

FIG.5

FIG.6

**FIG.7**

**FIG.8**

FIG.9

FIG.10

20                    23              15

21

22

FIG.11

15

21    22         23

0 136 262

FIG.12

FIG.14

FIG.13

**FIG.16**

**FIG.15**

0 136 262